# EUROPEAN PATENT SPECIFICATION

(11) **EP 3 989 882 B1**
(45) Date of publication and mention of the grant of the patent: **27.11.2024**
(21) Application number: 20736472.0
(22) Date of filing: 23.06.2020
(51) Int. Cl.: A61F 2/30, A61L 27/56

(54) **PLUG-SHAPED IMPLANT FOR THE REPLACEMENT AND REGENERATION OF BIOLOGICAL TISSUE AND METHOD FOR PREPARING THE IMPLANT**
STOPFENFÖRMIGES IMPLANTAT ZUM ERSETZEN UND REGENERIEREN VON BIOLOGISCHEM GEWEBE UND VERFAHREN ZUR HERSTELLUNG DES IMPLANTATS
IMPLANT EN FORME DE BOUCHON POUR LE REMPLACEMENT ET LA RÉGÉNÉRATION D'UN TISSU BIOLOGIQUE ET PROCÉDÉ DE PRÉPARATION DE L'IMPLANT

(30) Priority: 27.06.2019 NL 2023397
(43) Date of publication of application: 04.05.2022
(73) Proprietor: Jointsphere B.V., 5232 AC 's-Hertogenbosch (NL)
(72) Inventor: HERMSEN, Egidius Gerardus Maria, 5611 CZ Eindhoven (NL); VAN BUUL, Everardus Johannes Hubertus, 5212 AR 's-Hertogenbosch (NL); MELSOM, Giles William, 6721 NP Bennekom (NL); FRANSEN, Petrus Mattheus Egidius Adrianus, 5241 MB Rosmalen (NL)
(74) Representative: Arnold & Siedsma
(86) International application number: PCT/NL2020/050411
(87) International publication number: WO 2020/263086

(56) References cited:
- WO-A1-2018/204315
- US-A1- 2005 112 397
- US-A1- 2009 164 014
- US-A1- 2011 153 028
- US-A1- 2016 128 843

## Description

### TECHNICAL FIELD OF THE INVENTION

The invention relates to an implant for the replacement and regeneration of biological tissue in the shape of a plug. The invention in particular relates to an implant for the replacement and regeneration of an osteochondral structure in the shape of a plug. The invention further relates to a method for the preparation of the implant.

### BACKGROUND OF THE INVENTION

An osteochondral structure refers to a structure comprising cartilage and bone. Typical osteochondral structures can be found in the thighbone (femur), shinbone (tibia), and kneecap (patella). Such structures fit tightly together and move smoothly because the bone surface is covered with a relatively thick layer of articular (hyaline) cartilage. An (osteo)chondral defect is any type of damage to articular cartilage and optionally to underlying (subchondral) bone. Usually, (osteo)chondral defects appear on specific weight-bearing spots at the ends of the thighbone and shinbone and the back of the kneecap for instance. They may range from roughened cartilage, small bone and cartilage fragments that hinder movement, to complete cartilage loss.

Trauma of joint surfaces is common in young active people practicing sports, or as a sequel to accidents. Lesions may comprise the cartilage layer only, but often the underlying subchondral bone too. Articular cartilage has a very low tendency for healing and the repair tissue is qualitatively inferior to the original tissue. This invariably leads to the formation of osteoarthritis (OA) over the years, which is a major cause of disability and loss of quality of life in elderly people. The standard treatment for this condition is ultimately joint replacement by artificial joints. Whilst clinically effective, the non-biological implants do not last longer than 10-20 years and revision surgery is much less effective and very costly. For this reason, much research is dedicated to developing biological regenerative therapies that would be life-long lasting. However, despite promising in vitro results, until now not a single solution has proven to be more effective than the current standard of care over a longer period in real life conditions.

Because the cartilage layer lacks nerve fibers, patients are often not aware of the severity of the damage. During the final stage, an affected joint consists of bone rubbing against bone, which leads to severe pain and limited mobility. By the time patients seek medical treatment, surgical intervention may be required to alleviate pain and repair the cartilage damage. Implants have been developed for the joint in order to avoid or postpone such surgical interventions. These may be implanted in a bone structure at an early stage of cartilage damage, and may thus be provided for preventive treatment, in order to avoid unnoticed degeneration of the joint.

A number of treatments is available to treat articular cartilage damage in joints, such as the knee, starting with the most conservative, non-invasive options and ending with total joint replacement if the damage has spread throughout the joint. Currently available treatments include anti-inflammatory medications in the early stages. Although these may relieve pain, they have limited effect on arthritis symptoms and further do not repair joint tissue. Cartilage repair methods, such as arthroscopic debridement, attempt to at least delay tissue degeneration. These methods however are only partly effective at repairing soft tissue, and do not restore joint spacing or improve joint stability. Joint replacement (arthroplasty) is considered as a final solution, when all other options to relieve pain and restore mobility have failed or are no longer effective. While joint arthroplasty may be effective, the procedure is extremely invasive, technically challenging and may compromise future treatment options. Cartilage regeneration has also been attempted, more in particular by tissue-engineering technology. The use of cells, genes and growth factors combined with scaffolds plays a fundamental role in the regeneration of functional and viable articular cartilage. All of these approaches are based on stimulating the body's normal healing or repair processes at a cellular level. Many of these compounds are delivered on a variety of carriers or matrices including woven polylactic acid based polymers or collagen fibers. Despite various attempts to regenerate cartilage, a reliable and proven treatment does not currently exist for repairing defects to the articular cartilage.

Another standard of care consists of Microfracture (MFx) for smaller lesions (≤ 2 cm²) and Autologous Chondrocyte Implantation (ACI) for bigger lesions (> 2 cm²). The cartilaginous tissue regenerated with these techniques however is not able to withstand the biomechanical challenges in the joint and starts to degenerate within 18 months already. Substantial delay in joint replacement by artificial joints, let alone preventing it, therefore is not possible.

US2009/0164014A1 discloses a biodegradable osteochondral implant comprising a porous top and a porous bottom section separated by a barrier impermeable to certain agents

### SUMMARY OF THE INVENTION

It is an object of the present invention to provide an implant for the replacement and regeneration of biological tissue in the shape of a plug having improved load distribution as well as cartilage regenerating properties. Another aim is to provide such a plug-shaped implant for the replacement and regeneration of an osteochondral structure. Yet another aim is to provide a method for the preparation of the implant. The invention further aims to provide an implant which is able to repair articular cartilage lesions in a durable fashion, and which at least postpones and, preferably, prevents joint replacement by artificial joints.

The above and other aims are provided by an implant according to the invention as defined in claim 1.

With a substantially non-porous material in the context of the present invention is meant a material having a porosity of less than 20 %, relative to the total volume of the material, preferably up to 10%, more preferably up to 5%, and more preferably still up to 1% of the total volume of the material. A porous material comprises pores, which are defined as minute openings. The pores may be micropores, having a diameter of less than 1 mm, and may be macropores, having a diameter of greater than 1 mm. The pores may be interconnected, which is preferred, and which means that pores are internally connected or there is continuity between parts or elements. A non-porous material in the context of the present invention does not mean a material that is impermeable to molecules of any size, and some small molecules may indeed be able to pass through the non-porous material. Rather, a non-porous material in the context of the present invention represents a material that is impermeable to synovial fluid and/or blood.

Pore sizes in the porous parts of the implant may be chosen from 100-1000 micron, more preferably from 100-500 micron, and most preferably from 300-500 micron.

The materials used in the invented implant are preferably biocompatible, by which is meant that these materials are capable of coexistence with living tissues or organisms without causing harm to them. Further, the implant in accordance with the invention is substantially non-biodegradable and combines cartilage replacement with cartilage regeneration. With a non-biodegradable material in the context of the present invention is meant a material that is not broken down into less complex compounds or compounds having fewer carbon atoms by the environment of the implanted implant. The weight-average molecular weight of a substantially non-biodegradable material is reduced by at most 20%, relative to the original weight-average molecular weight after one year of implantation, more preferably at most 10%, still more preferably at most 5%, and more preferably still at most 1%.

The base section of the plug-shaped implant functions as a bone anchor, whereas the combination of middle and top sections functions as partial replacement for the damaged cartilage and as scaffold for cartilage regeneration. In the plug-shaped implant, the top section refers to the section that is closest to the cartilage phase, when implanted. The base section refers to the section that is furthest from the cartilage phase, when implanted. The middle section is situated in between the top and base sections.

The cross-section of the plug-shaped implant through a horizontal or a vertical plane may have any suitable shape. The cross-section may be circular, square or may be polygonal, such as hexagonal, octagonal, or decagonal. In some embodiments, the plug-shaped implant may be tapered such that it is shaped as a truncated cone structure. Preferably, the implant has a smaller cross-section at the base section than at the top section. The cross-section (or diameter in case of a cylindrical implant) may vary continuously between the base and top section, or may show discontinuities, for instance at the interface between sections.

When the implant has a tapered profile, the angle of the taper is preferably between 1° and 45°. In some embodiments, the taper is between about 3° and 30°, more preferably between 5° and 30°, even more preferably between 10° and 15°. A tapered profile may facilitate insertion of the implant into an osteochondral defect and may further reduce possible damage to host tissue.

A useful embodiment of the invention provides an implant, wherein the base section comprises a core of non-porous polyaryletherketone polymer and a, preferably circumferential, shell of porous polyaryletherketone polymer, wherein the shell has a thickness that is less than 10% of a largest diameter of the base section. Other useful embodiments provide an implant wherein the (circumferential) shell has a thickness of less than 9%, of less than 8%, of less than 7%, of less than 6%, of less than 5%, of less than 4%, of less than 3% , of less than 2%, or of less than 1% of a largest diameter of the base section. Alternatively, the cross-sectional area of the (circumferential) shell covers at most 35% of a largest cross-sectional area of the base section. Other useful embodiments provide an implant wherein the cross-sectional area of the circumferential shell is less than 30%, less than 25%, less than 20%, less than 15%, less than 10%, less than 5%, less than 3% , or less than 1% of a largest cross-sectional area of the base section.

Another embodiment of the invention provides an implant, wherein the base section extends between a top surface and a bottom surface, and comprises a layer of porous polyaryletherketone polymer, wherein the layer is adjacent to the top surface and has a thickness that is less than 10% of a largest height of the base section, and wherein pores of the polyaryletherketone polymer in the layer comprise a biocompatible elastomeric material that is the same as the material of the middle section, preferably all pores. In other embodiments, the layer that is adjacent to the top surface has a thickness of less than 10%, of less than 8%, of less than 6%, of less than 5%, of less than 4%, of less than 3%, of less than 2%, or of less than 1% of a largest height of the base section. All the above embodiments may improve the adhesion of the middle section (and top section) to the base section to varying degrees. At the same time, the mechanical properties of the base section, and the support offered by the base section to the implant, remain at an adequate level.

In another embodiment of the invention, the top surface of the base section of the implant comprises irregularities or undulations. Irregularities may for instance comprise ridges having a saw-toothed shape. Undulations may be irregular or regular, such as those having a sinusoidal shape.

Another useful embodiment relates to an implant, wherein the base section comprises a centrally located cavity that comprises a biocompatible elastomeric material. Such a cavity may further improve the adhesion of the middle section (and top section) to the base section. The cavity may be cylindrical, or its cross-section may be square, or polygonal. The walls of the cavity may also be provided with irregularities or undulations, or may comprise sections of a larger cross-sectional area than its average cross-sectional area. Several of such cavity sections may be provided at different heights of the base section to form mechanical locking structures.

A preferred embodiment of the invention provides an implant, wherein the base section comprises a non-porous polyaryletherketone polymer, and, more preferably, wherein the base section in combination comprises the central cavity.

In order to further improve the adhesion between the base section and the middle (and top) section, an embodiment of the invention provides an implant, wherein the base section further comprises a phosphate mineral comprising an apatite, more preferably a hydroxylapatite, a fluorapatite and/or a chlorapatite, most preferably a hydroxylapatite. The phosphate mineral may be provided at an outer surface of the base section, or may be provided within pores of the base section.

Yet another embodiment provides an implant, wherein the base section comprises an outer surface having irregularities or undulations. Such outer surface irregularities may for instance comprise ridges having a saw-toothed shape, for instance extending circumferentially over (part of) the outer surface of the base section. Undulations may be irregular or regular, such as those having a sinusoidal shape. The undulations may likewise extend circumferentially over (part of) the outer surface of the base section. Irregularities and undulations may be provided by casting the materials in a suitably profiled mold, or, alternatively, may be provided by mechanical machining, for instance by rotary milling of a molded implant.

The polyaryletherketone (PAEK) polymer of the base section comprises a semi-crystalline thermoplastic polymer containing alternately ketone (R-CO-R) and ether groups (R-O-R). The linking group R between the functional groups comprises a 1,4-substituted aryl group. The PAEK polymer used in the base section may *inter alia* comprise PEK (polyetherketone), PEEK (polyetheretherketone), PEKK (polyetherketoneketone), PEEKK (polyetheretherketoneketone) and PEKEKK (polyetherketoneetherketoneketone). Due to its excellent resistance to hydrolysis, the polyaryletherketone polymer of the base section is advantageously used in the invented implant. It does not break down when sterilized, nor when implanted in the body for an extended time. It also turns out to bond particularly well to the elastomeric material of the middle and top sections. The polyaryletherketone polymer of the base section may be used as such, or, in an embodiment, may comprise a reinforcing material selected from the group consisting of fibrous or particulate polymers and/or metals.

According to the invention, the material of the middle and top section comprises the same thermoplastic elastomeric material. By this is meant that at least its building blocks are chemically the same. As mentioned herein below, some physical properties may differ, for instance their weight averaged molecular weight. In a particularly suitable embodiment, the thermoplastic elastomeric material comprises a linear block copolymer comprising urethane and urea groups, and is substantially free of an added peptide compound having cartilage regenerative properties. It has surprisingly been found that the implant of the invention is able to regenerate cartilage tissue, thus avoiding the use of any functional compound exhibiting cartilage regenerative properties. In particular, it has been found that the implant according to this embodiment does not need the use of peptides, for instance those comprising an RGD-sequence. These compounds have been said to enable binding integrin's and thereby stimulating cell adhesion. Preferably, the thermoplastic elastomeric material is substantially free of any added compound having cartilage regenerative properties.

The thermoplastic elastomeric material of the implant according to an embodiment of the invention comprises a linear block (or segmented) copolymer. Such a copolymer comprises 'hard' crystallized blocks of polyurethane and/or polyurea segments, and may also comprise 'hard' crystallized blocks of polyester and/or polyamide between 'soft' blocks. At room temperature, the low melting 'soft' blocks may be incompatible with the high melting 'hard' blocks, which induces phase separation by crystallization or liquid-liquid demixing. These copolymers exhibit reversible physical crosslinks that originate from crystallization of the 'hard' blocks of the segmented copolymer. The thermoplastic elastomers may be formed into any shape at higher temperatures, more in particular at temperatures above the melting point of the 'hard' blocks. On the other hand, the thermoplastic elastomers provide mechanical stability and elastic properties at low temperatures, i.e. at typical body temperatures. This makes these materials particularly suitable as replacement material for human or animal cartilage.

In another preferred embodiment of the invented implant, the thermoplastic elastomeric material further comprises carbonate groups. This embodiment has proven to be beneficial in that its mechanical properties are well adapted to the mechanical properties of human or animal cartilage. Surprisingly, regeneration of cartilage is improved when using this embodiment in an implanted implant.

A particularly preferred embodiment of the invention provides an implant, wherein the thermoplastic elastomeric material comprises a poly-urethane-bisurea-alkylenecarbonate, more preferably a poly-urethane-bisurea-hexylenecarbonate.

The constituents of the thermoplastic elastomer may generally comprise three building blocks: a long-chain diol, for example with a polyether, polyester or polycarbonate backbone, a bifunctional di-isocyanate, and, finally, a chain extender, such as water, another (sometimes short-chain) diol, or a diamine. The latter chain extender is preferred since this leads to bisurea units in the thermoplastic elastomer.

An embodiment of the implant wherein the thermoplastic elastomeric material is aliphatic is preferred. This means that all building blocks of the thermoplastic elastomer are devoid of aromatic groups and contain aliphatic groups only. The thermoplastic elastomer of the invention may be prepared in a one pot procedure, in which a long-chain diol is first reacted with an excess of a di-isocyanate to form an isocyanate-functionalized prepolymer.

The latter is subsequently reacted with a chain extender, such as the preferred diamine, which results in the formation of a higher molecular weight thermoplastic elastomeric polymer containing urethane groups. If a diamine is used as the chain extender, the thermoplastic elastomer will also contain bisurea groups, which is preferred.

The synthetic procedure to prepare the thermoplastic elastomers may lead to a distribution in the 'hard' block lengths. As a result, the phase separation of these block copolymers may be incomplete, in that part of the 'hard' blocks, in particular the shorter ones, are dissolved in the soft phase, causing an increase in the glass transition temperature. This is less desired for the low temperature flexibility and elasticity of the thermoplastic elastomeric material of the top and middle sections. The polydispersity in 'hard' blocks shows as a broad melting range, and a rubbery plateau in dynamic mechanical thermal analysis (DMTA) that is dependent on temperature. Preferred embodiments therefore comprise elastomeric block copolymers containing 'hard' blocks of substantially uniform length. These may be prepared by fractionation of a mixture of 'hard' block oligomers, and subsequent copolymerization of the uniform 'hard' block oligomers of a specific length (or length variation) with the prepolymer, mentioned above.

Although the thermoplastic elastomers may be prepared by a chain extension reaction of an isocyanate-functionalized prepolymer with a diamine, they may also be prepared by a chain extension reaction of an amine-functionalized prepolymer with a di-isocyanate. Examples of suitable, commercially available diamines and di-isocyanates include alkylene diamines and/or di-isocyanates, arylene diamines and/or di-isocyanates. Amine-functionalized prepolymers are also commercially available, or can be prepared from (readily available) hydroxy functionalized prepolymers by cyanoethylation followed by reduction of the cyano-groups, by Gabriel synthesis (halogenation or tosylation followed by modification with phthalimide, and finally formation of the primary amine by deprotection of the phthalimide group) or by other methods that are known in the art. Isocyanate-functionalized prepolymers can be prepared by reaction of hydroxy functionalized prepolymers with di-isocyanates, such as for example isophorone di-isocyanate (IPDI), 1,4-diisocyanato butane, 1,6-diisocyanato hexane or 4,4'-methylene bis(phenyl isocyanate). Alternatively, isocyanate-functionalized prepolymers can be prepared from amine-functionalized prepolymers, for example by reaction with di-tert-butyl tricarbonate. Hydroxy-functionalized prepolymers of molecular weights typically ranging from about 500 g/mol to about 5000 g/mol of all sorts of compositions are also advantageously used. Examples include prepolymers of polyether's, such as polyethylene glycols, polypropylene glycols, poly(ethylene-co-propylene) glycols and poly(tetrahydrofuran), polyesters, such as poly(caprolactone)s or polyadipates, polycarbonates, polyolefins, hydrogenated polyolefins such as poly(ethylene-butylene)s, and the like. Polycarbonates are preferred.

The implant is preferably used without any means of attachment and remains in the osteochondral structure by its geometry and the surrounding tissue structure. The implant may be used in the knee, but may also be used for other joints, such as a temporal-mandibular joint, an ankle, a hip, a shoulder, and the like.

The thermoplastic elastomer used in the top and middle sections of the implant is particularly advantageous since it allows adapting its mechanical properties to those of human and animal cartilage. In an embodiment of the invention, an implant may be provided wherein the elastomeric material of the middle section has an elastic modulus at room temperature of less than 10 MPa, more preferably of less than 8 MPa, of less than 7 MPa, of less than 6 MPa, of less than 5 MPa, of less than 4 MPa, of less than 3 MPa, or of less than 2 MPa.

In the context of the present application, room temperature is meant to be a temperature in the range of 20-30°C, more preferably 25°C.

Likewise, preferred embodiments of the implant comprise a top section wherein the porous elastomeric material of the top section has an elastic modulus at room temperature of less than 80% of the elastic modulus of the elastomeric material of the middle section, more preferably of less than 50%, even more preferably of between 10-50%, even more preferably of between 15-40%, and most preferably of between 20-30% of the elastic modulus of the elastomeric material of the middle section. Such a reduced elastic modulus may be achieved by modifying the porosity of the material of the middle section, or by modifying physical properties of the material in the middle section through changing its weight average molecular weight for instance.

The porosity of the elastomeric material of the top section may be chosen within a broad range. Preferred porosities of the elastomeric material of the top section are selected from 20-80% by volume, more preferably from 30-70% by volume, even more preferably from 40-60% by volume, and most preferably from 45-55% by volume.

A useful embodiment of the invention provides an implant, wherein the middle section comprises a core of non-porous elastomeric material and a, preferably circumferential, shell of porous elastomeric material, wherein the shell has a thickness that is less than 10% of a largest diameter of the middle section. Other useful embodiments provide an implant wherein the (circumferential) shell has a thickness of less than 9%, of less than 8%, of less than 7%, of less than 6%, of less than 5%, of less than 4%, of less than 3% , of less than 2%, or of less than 1% of a largest diameter of the middle section. The largest diameter is for instance appropriate in an embodiment wherein the plug-shaped implant is tapered and has circular cross-sections. Alternatively, the cross-sectional area of the (circumferential) shell covers at most 35% of a largest cross-sectional area of the middle section. Other useful embodiments provide an implant wherein the cross-sectional area of the (circumferential) shell is less than 30%, less than 25%, less than 20%, less than 15%, less than 10%, less than 5%, less than 3%, or less than 1% of a largest cross-sectional area of the middle section. The largest cross-sectional area is for instance appropriate in an embodiment wherein the plug-shaped implant is tapered.

Embodiments having the above-disclosed preferred combinations of mechanical properties of the top and middle section tend to promote regeneration of cartilage. This is believed to be due to a favorable stress (re)distribution of the osteochondral structure including the implant during (dynamic) loading.

The height of the plug-shaped implant may be chosen according to the specific application in the body. Heights may vary from 3 to 18 mm for instance. According to a useful embodiment of the invention, an implant is provided wherein a height of the base section, a height of the non-porous middle section, and a height of the porous top section are selected such that a top surface of the implant comes to lie below a top surface of cartilage present on an osteochondral structure when implanted, preferably over a distance of between 0.1 - 1 mm. This embodiment promotes growing cartilage tissue into, but also onto the top section, whereby a strong fixation is built between the top section and the newly formed cartilage. It has turned out that cartilage cells from the host cartilage have a strong affinity for the segmented elastomer of the top section, and therefore are prone to colonize the surface thereof to produce new hyaline cartilage tissue on top of the implant.

Another embodiment provides an implant wherein a height of the base section, a height of the non-porous middle section, and a height of the porous top section are selected such that a bottom surface of the middle section comes to lie about level with a bottom surface of cartilage present on an osteochondral structure when implanted.

Yet another embodiment of the invention provides a top section, a top surface of which is slightly curved. Preferred radii of curvature of the top surface of the top section in a sagittal plane are selected to range from 15 - 150 mm, more preferably from 17 - 125 mm, even more preferably from 19 - 100 mm, even more preferably from 21 - 75 mm, even more preferably from 23 - 50 mm, and most preferably from 25 - 30 mm. This embodiment may regenerate a new cartilage layer on the top surface of the top section of the implant of about equal thickness across the top surface. The result may be a radius of a top surface of the regenerated cartilage that is about the same as the radius of the surrounding native cartilage layer next to the implant, thereby showing a continuity in radius. The top surface of the top section of the implant may also be curved in a medial-lateral plane, preferably with a radius of curvature with the ranges disclosed above for the sagittal plane. In a practical embodiment, the top surface of the top section of the implant has a radius of curvature that is equal in the sagittal and the medial-lateral plane. This embodiment thus comprises a spherical top surface.

Another aspect of the invention provides a method for the preparation of the implant as defined in claim 14.

Another embodiment of the invention provides a method wherein after step b) the mold is opened and additional granules of the thermoplastic elastomeric material are added to the mold, and step b) is repeated. The amount of material added in the two-step embodiment of the method may be chosen within wide ranges. Increasingly good results are obtained when the ratio between the first addition and the second addition of granules of the thermoplastic elastomeric material is selected from 01:99 to 99:01, more preferably from 30:70 to 97:03, and most preferably from 70:30 to 95:05.

Another embodiment of the invention provides a method wherein the heating temperature of step b) is between 110°C and 225°C, more preferably between 120°C and 200°C, and most preferably between 130°C and 175°C. Preferred pressures at all cited temperature ranges are between 1.1 and 1.8 GPa, and more preferably between 1.2 and 1.6 GPa.

### BRIEF DESCRIPTION OF THE FIGURES

The invention will now be further elucidated by the following figures and examples, without however being limited thereto. In the figures:
Figures 1A to 1D show a schematic side view of four embodiments of an exemplary implant according to the present invention;
Figure 2A shows a schematic perspective view of a base section according to an embodiment of the invention;
Figure 2B shows a schematic cross-section of the embodiment of figure 2A;
Figures 2C and 2D show a schematic detailed view of parts B and C of the embodiment of figure 2B;
Figure 3 shows a schematic representation of a possible synthetic route to the thermoplastic polycarbonate material according to an embodiment of the invention;
Figure 4 shows a ¹H-NMR spectrum of the thermoplastic polycarbonate material according to an embodiment of the invention;
Figures 5A to 5C show DSC thermograms of the thermoplastic polycarbonate material according to an embodiment of the invention at different heating rates;
Figures 6A to 6C show a schematic representation of a defect in an osteochondral structure (6A), the osteochondral structure comprising an implant according to an embodiment of the invention (6B) and the same osteochondral structure after on-/ingrowth of cartilage (6C);Figures 7A to 7D show a schematic side view of four embodiments of an implant according to yet another embodiment of the present invention; and finally
Figures 8A to 8C show a schematic representation of a defect in an osteochondral structure (8A), the osteochondral structure comprising an implant according to another embodiment of the invention (8B) and the same osteochondral structure after on-/ingrowth of cartilage (8C).

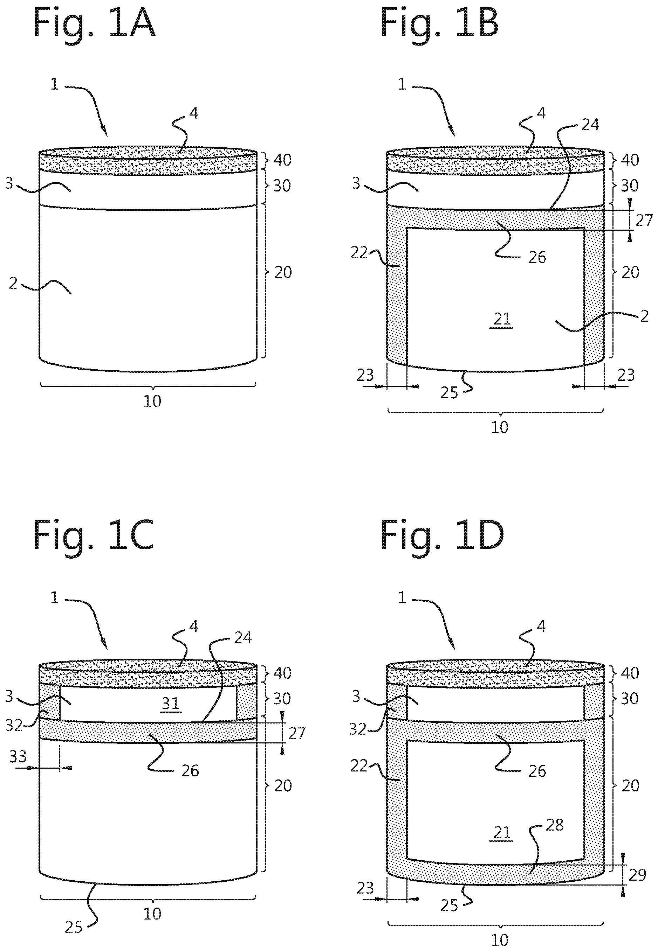

Referring to figure 1A, a side view of an embodiment of an exemplary implant according to the present invention is shown. The implant 1 in the shape of a plug comprises a base section 2, configured for anchoring in bone tissue, a middle section 3 configured for replacing cartilage tissue, and a top section 4 configured for growing cartilage tissue onto and into. The middle section 3 and top section 4 comprise the same thermoplastic elastomeric material. The thermoplastic elastomeric material in this embodiment comprises a poly-urethane-bisurea-hexylenecarbonate, the preparation and properties whereof will be elucidated further below. The top section 4 however comprises poly-urethane-bisurea-hexylenecarbonate in porous from, whereas the middle section 3 comprises the same poly-urethane-bisurea-hexylenecarbonate without any pores. The base section 2 comprises a non-porous polyaryletherketone polymer, which, in the embodiment shown is a non-porous PEKK polymer. The implant 1 is cylindrical and has a diameter 10 of 6 mm. The height 20 of the base section 2, the height 30 of the middle section 3, and the height 40 of the top section 4 add up to a total height of 6 mm.

Figure 1B schematically represents a side view of another embodiment of an implant according to the present invention. The embodied implant 1 in the shape of a plug again comprises a base section 2, configured for anchoring in bone tissue, a middle section 3 configured for replacing cartilage tissue, and a top section 4 configured for growing cartilage tissue onto and into. The middle section 3 and top section 4 comprise the same poly-urethane-bisurea-hexylenecarbonate material, which is porous in the top section 4, and non-porous in the middle section 3. The base section 2 comprises a substantially non-porous PEKK polymer with a porosity of less than 20 %, relative to the total volume of the PEKK polymer. The base section 2 of this embodiment in particular comprises a core 21 of non-porous PEKK polymer and a circumferential shell 22 of porous PEKK polymer. The shell 22 has a thickness 23 of about 8% of the diameter 10 of the base section 2 (and implant 1). The base section 2 further extends between a top surface 24 and a bottom surface 25, and comprises a layer 26 of porous PEKK polymer, which layer 26 is adjacent to the top surface 24 and has a thickness 27 of about 8% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. A method for manufacturing the implant will be elucidated further below. As with the embodiment of figure 1A, the implant 1 is cylindrical and has a diameter 10 of 6 mm. The height 20 of the base section 2, the height 30 of the middle section 3, and the height 40 of the top section 4 add up to a total height of 6 mm.

Figure 1C schematically represents a side view of yet another embodiment of an implant according to the present invention. The embodied implant 1 in the shape of a plug again comprises a base section 2, configured for anchoring in bone tissue, a middle section 3 configured for replacing cartilage tissue, and a top section 4 configured for growing cartilage tissue onto and into. The middle section 3 and top section 4 comprise the same poly-urethane-bisurea-hexylenecarbonate material, which is porous in the top section 4, and substantially non-porous in the middle section 3. The base section 2 comprises a substantially non-porous PEKK polymer with a porosity of less than 20 %, relative to the total volume of the PEKK polymer. The base section 2 of this embodiment in particular extends between a top surface 24 and a bottom surface 25, and comprises a layer 26 of porous PEKK polymer, which layer 26 is adjacent to the top surface 24 and has a thickness 27 of about 8% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. The middle section 3 of this embodiment in particular comprises a core 31 of non-porous poly-urethane-bisurea-hexylenecarbonate polymer and a circumferential shell 32 of porous poly-urethane-bisurea-hexylenecarbonate polymer. The shell 32 has a thickness 33 of about 8% of the diameter 10 of the middle section 3 (and implant 1). The base section 2 further extends between a top surface 24 and a bottom surface 25, and comprises a layer 26 of porous PEKK polymer, which layer 26 is adjacent to the top surface 24 and has a thickness 27 of about 8% of the height 20 of the base section 2. The dimensions and shape are the same as in the embodiments of figures 1A and 1B.

Figure 1D schematically represents a side view of yet another embodiment of an implant according to the present invention. The embodied implant 1 in the shape of a plug corresponds to the one shown in figure 1C. In addition, the middle section 3 of this embodiment now has a circumferential shell 32 of porous poly-urethane-bisurea-hexylenecarbonate polymer having a thickness 33 of about 10% of the diameter 10 of the middle section 3 (and implant 1). Further, the base section 2 comprises a layer 26 of porous PEKK polymer, which layer 26 is adjacent to the top surface 24 and has a thickness 27 of about 5% of the height 20 of the base section 2. The pores of the PEKK polymer in the layer 26 comprise the biocompatible poly-urethane-bisurea-hexylenecarbonate which originates from the middle section 3 and has infiltrated the pores of the PEKK polymer in the layer 26 during manufacturing. The base section 2 further comprises a core 21 of non-porous PEKK polymer and a circumferential shell 22 of porous PEKK polymer. The shell 22 has a thickness 23 of about 5% of the diameter 10 of the base section 2 (and implant 1). Finally, the base section 2 also comprises a layer 28 of porous PEKK polymer, which layer 28 is adjacent to the bottom surface 25 and has a thickness 29 of about 5% of the height 20 of the base section 2. The dimensions and shape are the same as in the embodiments of figures 1A to 1C.

Please note that in figures 1B, 1C, and 1D the circumferential shells (22, 32) are shown in cross-section to show their respective thicknesses (23, 33). In a side view, they would extend over the complete diameter 10 of the implant 1.

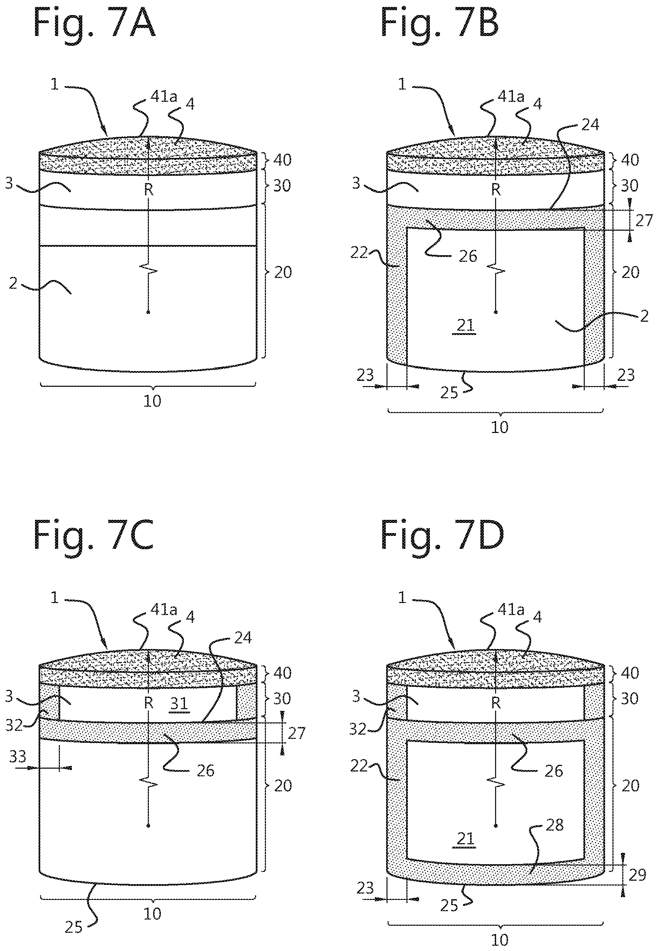

Referring to figure 7A, a side view of another embodiment of the implant according to the present invention is shown. The implant 1 in the shape of a plug comprises the same materials and sections as shown in figure 1A. The dimensions of the implant of figure 7A are the same as those of the implant of figure 1A with one exception. Instead of having a flat top surface 41 of the top section 4 (and the implant 1), as in figure 1A, the top surface 41a of the top section 4 is spherical with a radius of curvature R of about 28 mm (not drawn to scale).

Referring to figure 7B, a side view of another embodiment of the implant according to the present invention is shown. The implant 1 in the shape of a plug comprises the same materials and sections as shown in figure 1B. The dimensions of the implant of figure 7B are the same as those of the implant of figure 1B with one exception. Instead of having a flat top surface 41 of the top section 4, as in figure 1B, the top surface 41a of the top section 4 is spherical with a radius of curvature R of about 28 mm (not drawn to scale). Referring to figure 7C, a side view of another embodiment of the implant according to the present invention is shown. The implant 1 in the shape of a plug comprises the same materials and sections as shown in figure 1C. The dimensions of the implant of figure 7C are the same as those of the implant of figure 1C with one exception. Instead of having a flat top surface 41 of the top section 4, as in figure 1C, the top surface 41a of the top section 4 is spherical with a radius of curvature R of about 28 mm (not drawn to scale).

Referring to figure 7D, a side view of another embodiment of the implant according to the present invention is shown. The implant 1 in the shape of a plug comprises the same materials and sections as shown in figure 1D. The dimensions of the implant of figure 7D are the same as those of the implant of figure 1D with one exception. Instead of having a flat top surface 41 of the top section 4, as in figure 1D, the top surface 41a of the top section 4 is spherical with a radius of curvature R of about 28 mm (not drawn to scale).

Again note that in figures 7B, 7C, and 7D the circumferential shells (22, 32) are shown in cross-section to show their respective thicknesses (23, 33). In a side view, they would extend over the complete diameter 10 of the implant 1 (not drawn to scale).

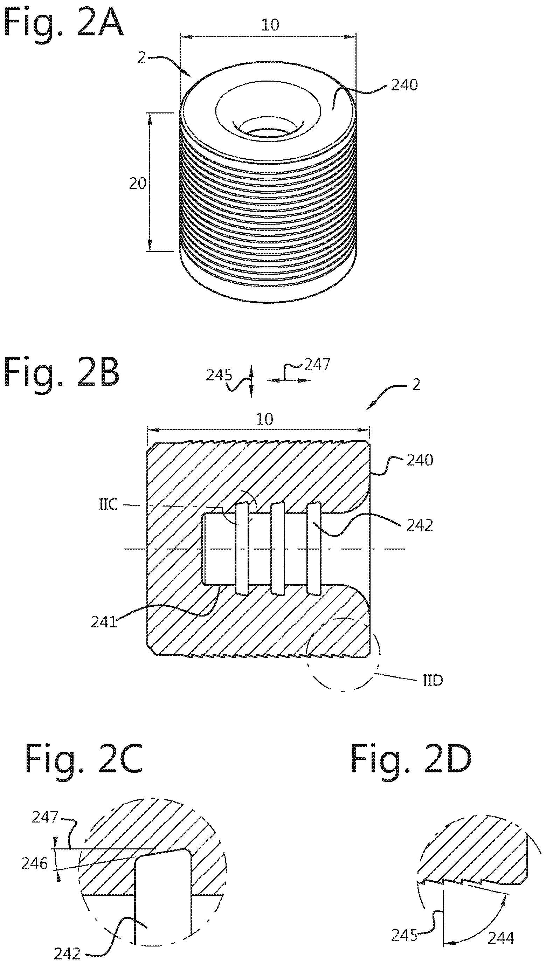

Referring to figures 2A to 2D, an embodiment of a base section 2 of the invented implant 1 is schematically shown. The base section 2 shown is essentially cylindrical-shaped with a diameter 10, and a height 20. The top surface 24 of the base section has a circumferential flat rim part 240 that gradually extends into a centrally located cavity 241. The cavity 241 is provided with locking parts 242 that have a larger diameter than the diameter of the cavity 241. A shown in detail in figure 2C, the locking parts 242 of the cavity 241 are disk-shaped whereby the outer rim of the disk makes an angle 246 with the longitudinal direction 247 of the base section 2 of between 1° and 20°, more preferably between 5° and 15°. The cavity 241 (and parts 242) during manufacturing of the implant fills with part of the biocompatible elastomeric material to provide an adequate locking of the middle section 3 to the base section 2. As discussed above, the base section 2 comprises a PEKK polymer which may be non-porous or substantially non-porous, the latter embodiment including the examples disclosed above. The base section 2 is further seen to comprise an outer surface having irregularities or undulations. In the present embodiment, these comprise circumferential ridges 243 which, in cross-section, are saw-tooth-shaped, as shown in detail in figure 2D. The angle 244 under which the saw-tooth flanks extend with respect to the transverse direction 245 of the base section 2, is preferably between 70° and 85°, more preferably between 75° and 80°.

### PREPARATION OF THE ELASTOMERIC MATERIAL OF THE TOP AND MIDDLE SECTION

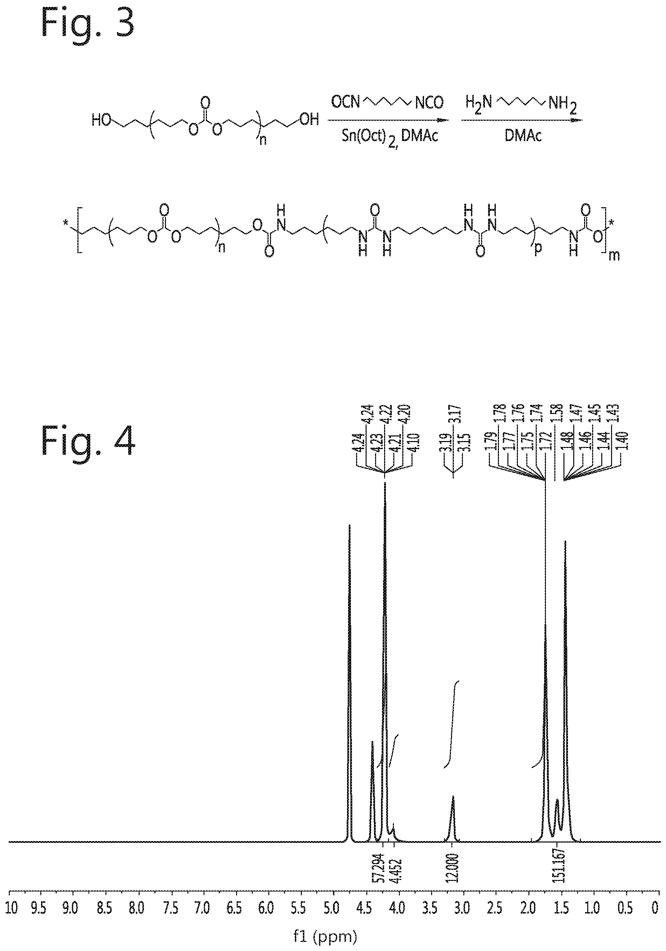

The aliphatic poly-urethane-urea-hexylene carbonate biomaterial of the middle section 3 and the top section 4 was manufactured as follows (with reference to figure 3). Poly(hexylene carbonate) diol (23.9 g, 11.9 mmol) was weighed in a 500 mL 3-necked flask and dried by heating to 75 °C overnight under vacuum, after which it was allowed to cool to room temperature. Under an argon atmosphere, 1,6-diisocyanatohexane (4.1 g, 23.9 mmol), DMAc (20 mL) and a drop of Sn(II)bis(2-ethylhexanoate) were added, after which the mixture was heated and stirred for 3 hours upon which the viscosity increased. The mixture was allowed to cool to room temperature, was diluted with DMAc (100 mL) and a solution of 1,6-diaminohexane (1.4 g, 11.9 mmol) in DMAc (50 mL) was added at once under thorough mixing. A gel was immediately formed upon addition and mixing. The mixture was further diluted with DMAc (150 mL) and was heated in an oil bath of 130 °C to acquire a homogeneous viscous slurry. After cooling to room temperature, the mixture was precipitated in a water/brine mixture (2.75 L water + 0.25 L saturated brine) to yield a soft white material. This material was cut into smaller pieces and was stirred in a 1:5 mixture of methanol and water (3 L) for 64 hours. After decanting the supernatant, the resulting solid was stirred in a 2:1 mixture of methanol and water (0.75 L) for 6 hours. Decanting of supernatant, stirring in a 2:1 mixture of methanol and water (0.75 L) for 16 hours, decanting of the supernatant, and drying of the solid at 70 °C in vacuo yielded a flexible, tough elastomeric polymer.

¹H NMR spectroscopy was performed on the resulting polymer, using a Varian 200, a Varian 400 MHz, or a 400 MHz Bruker spectrometer at 298K. DSC was performed using a Q2000 machine (TA Instruments). Heating scan rates of 10 °C/min and 40 °C/min were used for the assessment of the melting temperature (Tm) and the glass transition temperature (Tg), respectively. The Tm was determined by the peak melting temperature and the Tg was determined from the inflection point.

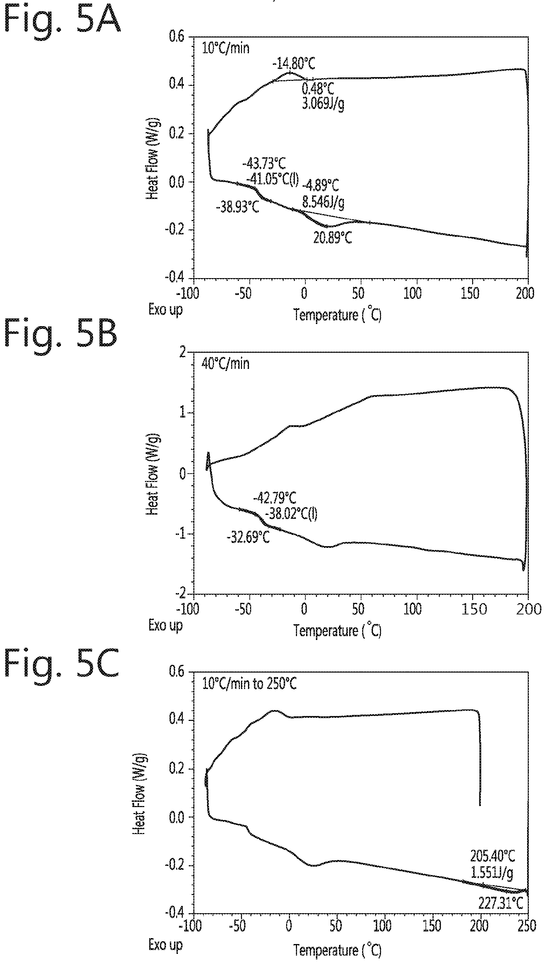

All reagents, chemicals, materials, and solvents were obtained from commercial sources and were used without further purification. The used poly(hexylene carbonate) diol had an average molecular weight of approximately 2 kg/mol. Figures 4 and 5 show the ¹H NMR spectrum and DSC thermograms of the obtained polymer, respectively. The ¹H NMR spectrum results may be summarized as follows: ¹H NMR (400 MHz, HFIP-d2): δ = 4.23 (m, n*4H, n - 14.3), 4.10 (m, 4H), 3.17 (m, 12H), 1.87-1.32 (multiple signals for aliphatic CH2 methylenes) ppm. The average molecular weight of the repeating hard/soft block sections is about 2.5 kDa. The DSC results may be summarized as follows: DSC (10 °C/min, figure 5A): Tm (top) = 20.9 °C (soft block melt); DSC (40 °C/min, figure 5B): Tg = -38.0 °C. No second melting point for the hard block was observed up to 200 °C. However, in a final heating run up to 250 °C at 10 °C/min (figure 5C), a small and broad melting transition was observed at ca. 227 °C. In the DSC-diagrams, the endothermic melting peaks are plotted downwards, whereas the exothermic crystallizations are plotted upwards.

The non-porous aliphatic poly-urethane-urea-hexylene carbonate biomaterial had an elastic modulus according to ASTM D638 of 3.6 ± 0.03 MPa.

### PREPARATION OF BIOMATERIAL-CAPPED PEKK BONE ANCHORS

The implant 1 was manufactured by attaching the top and middle sections (4, 3) to a PEKK base section 2 which serves as bone anchor. In a method according to an embodiment of the invention, PEKK bone anchors were capped with the poly-urethane-urea-hexylene carbonate biomaterial by pressing small granules of the aliphatic polycarbonate polymer on top of and into the PEKK anchors. For this purpose, a custom press setup was used. Various temperatures (100 °C to about 150 °C), compressive forces (2 kN to about 4 kN) and methods have been tested. The best results were obtained using a two-step procedure, employing a temperature of 150 °C and using a compressive force of 40 kN (4 tons, or 4000 kg; corresponding to a pressure of 1.4 GPa). Lower temperatures than 150 °C seemed to give less homogenously pressed poly-urethane-urea-hexylene carbonate biomaterial layers (sections 3 and 4), while higher temperatures are less desired as the urea groups in the poly-urethane-urea-hexylene carbonate biomaterial may then degrade to some extent. In the first step, ca. 50 mg of the polymer 12 was pressed onto and into the PEKK bone anchor for 15 minutes, while in the second step, ca. 2 mg of polymer 12 was added to the setup and the sample was pressed for another 15 minutes under the same conditions (150 °C and 40 kN). The samples were subsequently removed from the compression setup and were then allowed to cool. After the second pressing step, the surface of the poly-urethane-urea-hexylene carbonate biomaterial layer (sections 3 and 4) on top of the base section 2 seemed to be substantially flat. The biomaterial was almost transparent and colorless. The edges of the biomaterial showed some fringes or frays, and these were removed using a scalpel.

A central hole (241, 242) of the base section 2 was about 4.5 mm deep and about 2 mm in diameter. The hole was substantially filled with the poly-urethane-urea-hexylene carbonate biomaterial, and the attachment of the biomaterial to the PEKK base section 2 seemed quite strong and robust. Removing the biomaterial from the PEKK base section by force, or loosening the connection at the PEKK-biomaterial interfaces, proved practically impossible. All used equipment and accessories that were intended to come into contact with the PEKK base section 2 and/or with the elastomeric biomaterial were rinsed with ethanol or isopropanol and were thereafter dried. After pressing, and cutting the frays, the PEKK-biomaterial plug implant was rinsed with isopropanol and dried. The plugs may also be produced in a sterilized environment, if needed.

As assessed by measuring, the PEKK base section was 6 mm in diameter and 6 mm tall (a height of 6 mm). The central cavity in the base section was about 2 mm in diameter and about 4.5 mm deep. The elastomeric biomaterial (the aliphatic polycarbonate) positioned onto the PEKK base section was about 6 mm in diameter and about 1 mm high. Accordingly, the total PEKK-biomaterial plug implant was about 7 mm tall.

The top section 4 was provided with pores by drilling holes in it with an average diameter of 300 micron, to a final porosity of 50 vol.%. The porous aliphatic poly-urethane-urea-hexylene carbonate biomaterial of the top section 4 had an elastic modulus according to ASTM D638 of 0.9 ± 0.2 MPa.

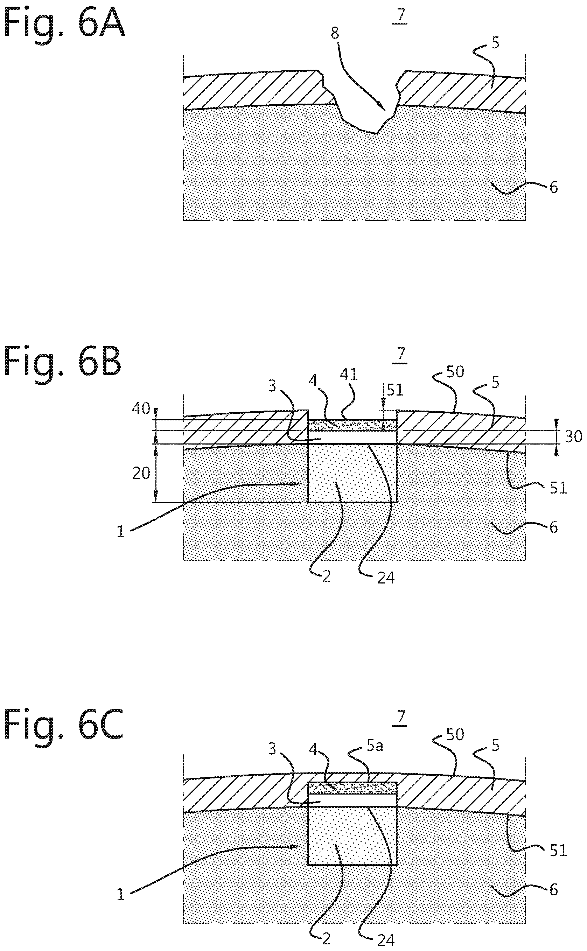

The implant 1 may be implanted into an osteochondral defect 8 as shown in figures 6A to 6C. In a typical method, a cartilage defect extending into the subchondral bone (figure 6 A) is drilled out and a plug-shaped implant 1 is implanted into the drilled hole under some pressure (`press fit'), as shown in figure 6B. Bone then grows onto, and in some embodiments into, the PEKK base section 2, anchoring the implant 1. Surrounding native cartilage 5 grows onto a top side 41 of the top section 4 and new cartilage 5a is generated on top of the implant 1, as shown in figure 6C. As is also shown in figure 6C, the height 20 of the base section 2, the height 30 of the non-porous middle section 3, and the height 40 of the porous top section 4 are selected such that a top surface 41 of the implant 1 comes to lie below a top surface 50 of cartilage 5 present on an osteochondral structure (5, 6) when implanted, preferably over a distance 51 of between 0.1 - 1 mm. In the present case, this distance was about 0.5 mm. The osteochondral structure (5, 6) comprises subchondral bone 6 and a cartilage layer 5 on top of it. A synovial cavity 7 is generally also present.

As also shown in figures 6B and 6C, the height 20 of the base section 2, the height 30 of the non-porous middle section 3, and the height 40 of the porous top section 4 are selected such that a bottom surface 24 of the middle section 3 (or top surface 24 of the base section 2) comes to lie about level with a bottom surface 51 of the cartilage layer 5 of the osteochondral structure (5, 6) when implanted.

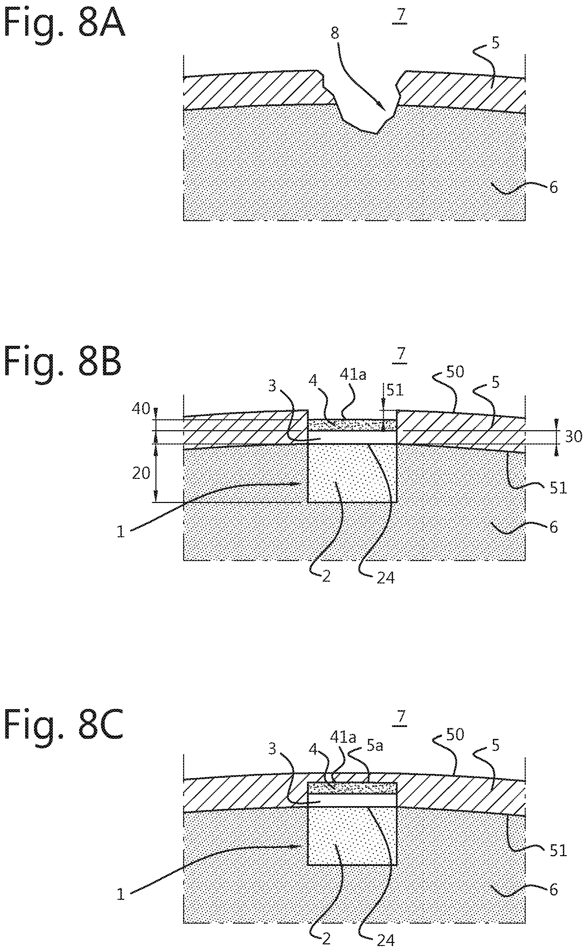

Finally, the implant according to the embodiment shown in figures 7A to 7D may also be implanted into an osteochondral defect 8 as shown in figures 8A to 8C. Due to a spherical top surface 41a of the top layer 4, this embodiment may regenerate a new cartilage layer 5a on the top surface 41a of the top section 4 of the implant 1 of about equal thickness across the top surface 41a. The result may be a radius of a top surface 50 of the regenerated cartilage 5a that is about the same as the radius of the surrounding native cartilage layer 5 next to the implant, thereby showing a continuity in radius.

It will be apparent that many variations and applications are possible for a skilled person in the field within the scope of the appended claims of the invention.

## Claims

1. An implant (1) for the replacement and regeneration of biological tissue in the shape of a plug, comprising a base section (2) configured for anchoring in bone tissue, a middle section (3) configured for replacing cartilage tissue, and a top section (4) configured for growing cartilage tissue onto and into, wherein the middle (3) and top section (4) comprise the same thermoplastic elastomeric material, which is porous in the top section (4), and non-porous in the middle section (3), **characterized in that** the base section (2) comprises a substantially non-porous polyaryletherketone polymer with a porosity of less than 20 %, relative to the total volume of the polyaryletherketone polymer.

2. Implant (1) according to claim 1, wherein the base section (2) comprises a core (21) of non-porous polyaryletherketone polymer and a circumferential shell (22) of porous polyaryletherketone polymer, wherein the shell (22) has a thickness that is less than 10% of a largest diameter of the base section (2).

3. Implant (1) according to claim 1 or 2, wherein the base section (2) extends between a top surface (24) and a bottom surface (25), and comprises a layer (26) of porous polyaryletherketone polymer, wherein the layer (26) is adjacent to the top surface (24) and has a thickness that is less than 10% of a largest height of the base section (2), and wherein the pores of the polyaryletherketone polymer in the layer (26) comprise a biocompatible elastomeric material that is the same as the material of the middle section (3).

4. Implant (1) according to any one of the preceding claims, wherein the base section (2) extends between a top surface (24) and a bottom surface (25) and wherein the top surface of the base section (2) comprises irregularities or undulations.

5. Implant (1) according to any one of the preceding claims, wherein the base section (2) comprises a centrally located cavity (241) that comprises a biocompatible elastomeric material.

6. Implant (1) according to any one of the preceding claims, wherein the base section (2) comprises a non-porous polyaryletherketone polymer.

7. Implant (1) according to any one of the preceding claims, wherein the base section (2) further comprises a phosphate mineral comprising an apatite, more preferably a hydroxylapatite, a fluorapatite and/or a chlorapatite, most preferably a hydroxylapatite.

8. Implant (1) according to any one of the preceding claims, wherein the porous thermoplastic elastomeric material of the top section (4) has an elastic modulus at room temperature of less than 80% of an elastic modulus of the thermoplastic elastomeric material of the middle section (3).

9. Implant (1) according to any one of the preceding claims, wherein the top section (4) has a slightly curved top surface, having a radius of curvature in a sagittal and/or a medial-lateral plane ranging from 15 mm to 150 mm.

10. Implant (1) according to any one of the preceding claims, wherein the thermoplastic elastomeric material comprises a linear block copolymer comprising urethane and urea groups, and is substantially free of an added peptide compound having cartilage regenerative properties.

11. Implant (1) according to claim 10, wherein the thermoplastic elastomeric material further comprises carbonate groups.

12. Implant (1) according to claim 11, wherein the thermoplastic elastomeric material comprises a poly-urethane-bisurea-alkylenecarbonate.

13. Implant (1) according to any one of the preceding claims, wherein the middle section (3) comprises a core (31) of non-porous elastomeric material and a circumferential shell (32) of porous elastomeric material, wherein the shell has a thickness that is less than 10% of a largest diameter of the middle section (3).

14. A method for the preparation of an implant in accordance with any one of the preceding claims, comprising:
a) providing in a mold at room temperature an assembly comprising a base section that comprises a substantially non-porous polyaryletherketone polymer with a porosity of less than 20 % relative to the total volume of the polyaryletherketone polymer; and granules of a thermoplastic elastomeric material on top of the base section;
b) closing the mold and heating the above assembly to a temperature of between 100°C and 250°C under a pressure of between 1 and 2 GPa, such that the thermoplastic elastomeric material melts and fuses with the base section; and
c) cooling the assembly to room temperature to consolidate the thermoplastic elastomeric material and opening the mold;
d) providing a top section of the thermoplastic elastomeric material with pores either before or after opening the mold.

15. A method according to claim 14, wherein after step b) the mold is opened and additional granules of the thermoplastic elastomeric material are added to the mold, and step b) is repeated.

## Patentansprüche

1. Implantat (1) für den Ersatz und die Regeneration von biologischem Gewebe in der Form eines Pfropfes, umfassend einen Basisabschnitt (2), der zum Verankern in Knochengewebe konfiguriert ist, einen Mittelabschnitt (3), der zum Ersetzen von Knorpelgewebe konfiguriert ist, und einen oberen Abschnitt (4), der zum Auf- und Einwachsen von Knorpelgewebe konfiguriert ist, wobei der Mittelabschnitt (3) und der obere Abschnitt (4) das gleiche thermoplastische Elastomermaterial umfassen, das in dem oberen Abschnitt (4) porös und in dem Mittelabschnitt (3) nicht porös ist, **dadurch gekennzeichnet, dass** der Basisabschnitt (2) ein im Wesentlichen nicht poröses Polyaryletherketonpolymer mit einer Porosität von weniger als 20 %, relativ zu dem Gesamtvolumen des Polyaryletherketonpolymers, umfasst.

2. Implantat (1) nach Anspruch 1, wobei der Basisabschnitt (2) einen Kern (21) aus nicht-porösem Polyaryletherketonpolymer und eine umlaufende Schale (22) aus porösem Polyaryletherketonpolymer umfasst, wobei die Schale (22) eine Dicke aufweist, die weniger als 10 % eines größten Durchmessers des Basisabschnitts (2) beträgt.

3. Implantat (1) nach Anspruch 1 oder 2, wobei sich der Basisabschnitt (2) zwischen einer oberen Oberfläche (24) und einer unteren Oberfläche (25) erstreckt und eine Schicht (26) aus porösem Polyaryletherketonpolymer umfasst, wobei die Schicht (26) an die obere Oberfläche (24) angrenzt und eine Dicke aufweist, die weniger als 10 % einer größten Höhe des Basisabschnitts (2) beträgt, und wobei die Poren des Polyaryletherketonpolymers in der Schicht (26) ein biokompatibles Elastomermaterial umfassen, das das gleiche wie das Material des Mittelabschnitts (3) ist.

4. Implantat (1) nach einem der vorstehenden Ansprüche, wobei sich der Basisabschnitt (2) zwischen einer oberen Oberfläche (24) und einer unteren Oberfläche (25) erstreckt und wobei die obere Oberfläche des Basisabschnitts (2) Unregelmäßigkeiten oder Wellen aufweist.

5. Implantat (1) nach einem der vorstehenden Ansprüche, wobei der Basisabschnitt (2) einen zentral eingerichteten Hohlraum (241) umfasst, der ein biokompatibles Elastomermaterial umfasst.

6. Implantat (1) nach einem der vorstehenden Ansprüche, wobei der Basisabschnitt (2) ein nicht-poröses Polyaryletherketonpolymer umfasst.

7. Implantat (1) nach einem der vorstehenden Ansprüche, wobei der Basisabschnitt (2) ferner ein Phosphatmineral umfasst, umfassend einen Apatit, stärker bevorzugt einen Hydroxylapatit, einen Fluorapatit und/oder einen Chlorapatit, am stärksten bevorzugt einen Hydroxylapatit.

8. Implantat (1) nach einem der vorstehenden Ansprüche, wobei das poröse thermoplastische Elastomermaterial des oberen Abschnitts (4) bei Raumtemperatur einen Elastizitätsmodul von weniger als 80 % des Elastizitätsmoduls des thermoplastischen Elastomermaterials des Mittelabschnitts (3) aufweist.

9. Implantat (1) nach einem der vorstehenden Ansprüche, wobei der obere Abschnitt (4) eine leicht gekrümmte obere Oberfläche aufweist, die einen Krümmungsradius in einer sagittalen und/oder medial-lateralen Ebene in einem Bereich von 15 mm bis 150 mm aufweist.

10. Implantat (1) nach einem der vorstehenden Ansprüche, wobei das thermoplastische Elastomermaterial ein lineares Blockcopolymer umfasst, umfassend Urethan- und Harnstoffgruppen, und im Wesentlichen frei von einer zugesetzten Peptidverbindung ist, die knorpelregenerierende Eigenschaften aufweist.

11. Implantat (1) nach Anspruch 10, wobei das thermoplastische Elastomermaterial ferner Carbonatgruppen aufweist.

12. Implantat (1) nach Anspruch 11, wobei das thermoplastische Elastomermaterial ein Polyurethan-Bisharnstoff-Alkylencarbonat umfasst.

13. Implantat (1) nach einem der vorstehenden Ansprüche, wobei der Mittelabschnitt (3) einen Kern (31) aus nicht-porösem Elastomermaterial und eine umlaufende Schale (32) aus porösem Elastomermaterial umfasst, wobei die Schale eine Dicke aufweist, die weniger als 10 % eines größten Durchmessers des Mittelabschnitts (3) beträgt.

14. Verfahren für die Herstellung eines Implantats nach einem der vorstehenden Ansprüche, umfassend:
a) Bereitstellen, in einer Form bei Raumtemperatur, einer Anordnung, umfassend einen Basisabschnitt, der ein im Wesentlichen nicht-poröses Polyaryletherketonpolymer mit einer Porosität von weniger als 20 % relativ zu dem Gesamtvolumen des Polyaryletherketonpolymers umfasst; und Granulat aus einem thermoplastischen Elastomermaterial auf dem Basisabschnitt;
b) Schließen der Form und derartiges Erhitzen der obigen Anordnung auf eine Temperatur zwischen 100°C und 250 °C unter einem Druck zwischen 1 und 2 GPa, dass das thermoplastische Elastomermaterial schmilzt und mit dem Basisabschnitt verschmilzt; und
c) Abkühlen der Anordnung auf Zimmertemperatur, um das thermoplastische Elastomermaterial zu verfestigen, und Öffnen der Form;
d) Versehen eines oberen Abschnitts des thermoplastischen Elastomermaterials mit Poren entweder vor oder nach dem Öffnen der Form.

15. Verfahren nach Anspruch 14, wobei nach Schritt b) die Form geöffnet wird und zusätzliches Granulat des thermoplastischen Elastomermaterials in die Form zugegeben wird und Schritt b) wiederholt wird.

## Revendications

1. Implant (1) en forme de bouchon pour le remplacement et la régénération de tissu biologique, comprenant une section de base (2) conçue pour s'ancrer dans un tissu osseux, une section médiane (3) conçue pour remplacer un tissu cartilagineux, et une section supérieure (4) conçue pour faire croître un tissu cartilagineux sur et dans celle-ci, dans lequel la section médiane (3) et la section supérieure (4) comprennent le même matériau élastomère thermoplastique, qui est poreux dans la section supérieure (4) et non poreux dans la section médiane (3), **caractérisé en ce que** la section de base (2) comprend un polymère de polyaryléthercétone sensiblement non poreux avec une porosité inférieure à 20 % par rapport au volume total du polymère de polyaryléthercétone.

2. Implant (1) selon la revendication 1, dans lequel la section de base (2) comprend un noyau (21) en polymère de polyaryléthercétone non poreux et une enveloppe circonférentielle (22) en polymère de polyaryléthercétone poreux, dans lequel l'enveloppe (22) a une épaisseur inférieure à 10 % d'un plus grand diamètre de la section de base (2).

3. Implant (1) selon la revendication 1 ou 2, dans lequel la section de base (2) s'étend entre une surface supérieure (24) et une surface inférieure (25), et comprend une couche (26) de polymère de polyaryléthercétone poreux, dans lequel la couche (26) est adjacente à la surface supérieure (24) et a une épaisseur inférieure à 10 % d'une plus grande hauteur de la section de base (2), et dans lequel les pores du polymère de polyaryléthercétone dans la couche (26) comprennent un matériau élastomère biocompatible qui est le même que le matériau de la section médiane (3).

4. Implant (1) selon l'une quelconque des revendications précédentes, dans lequel la section de base (2) s'étend entre une surface supérieure (24) et une surface inférieure (25) et dans lequel la surface supérieure de la section de base (2) comporte des irrégularités ou des ondulations.

5. Implant (1) selon l'une quelconque des revendications précédentes, dans lequel la section de base (2) comprend une cavité (241) située au centre qui comprend un matériau élastomère biocompatible.

6. Implant (1) selon l'une quelconque des revendications précédentes, dans lequel la section de base (2) comprend un polymère de polyaryléthercétone non poreux.

7. Implant (1) selon l'une quelconque des revendications précédentes, dans lequel la section de base (2) comprend en outre un minéral phosphaté comprenant une apatite, plus préférablement une hydroxylapatite, une fluorapatite et/ou une chlorapatite, le plus préférablement une hydroxylapatite.

8. Implant (1) selon l'une quelconque des revendications précédentes, dans lequel le matériau élastomère thermoplastique poreux de la section supérieure (4) a un module d'élasticité à température ambiante inférieur à 80 % d'un module d'élasticité du matériau élastomère thermoplastique de la section médiane (3).

9. Implant (1) selon l'une quelconque des revendications précédentes, dans lequel la section supérieure (4) présente une surface supérieure légèrement incurvée, avec un rayon de courbure dans un plan sagittal et/ou un plan médio-latéral compris entre 15 mm et 150 mm.

10. Implant (1) selon l'une quelconque des revendications précédentes, dans lequel le matériau élastomère thermoplastique comprend un copolymère bloc linéaire comprenant des groupes uréthane et urée, et est sensiblement dépourvu d'un composé peptidique ajouté ayant des propriétés de régénération du cartilage.

11. Implant (1) selon la revendication 10, dans lequel le matériau élastomère thermoplastique comprend en outre des groupes carbonates.

12. Implant (1) selon la revendication 11, dans lequel le matériau élastomère thermoplastique comprend un poly-uréthane-bisurée-alkylènecarbonate.

13. Implant (1) selon l'une quelconque des revendications précédentes, dans lequel la section médiane (3) comprend un noyau (31) en matériau élastomère non poreux et une enveloppe circonférentielle (32) en matériau élastomère poreux, dans lequel l'enveloppe a une épaisseur inférieure à 10 % d'un plus grand diamètre de la section médiane (3).

14. Procédé pour la préparation d'un implant selon l'une quelconque des revendications précédentes, comprenant :
a) la fourniture dans un moule à température ambiante d'un ensemble comprenant une section de base qui comprend un polymère de polyaryléthercétone sensiblement non poreux avec une porosité inférieure à 20 % par rapport au volume total du polymère de polyaryléthercétone ; et des granulés d'un matériau élastomère thermoplastique au-dessus de la section supérieure de la section de base ;
b) la fermeture du moule et le chauffage de l'ensemble ci-dessus à une température comprise entre 100 °C et 250 °C sous une pression comprise entre 1 et 2 GPa, de manière à ce que le matériau élastomère thermoplastique fonde et fusionne avec la section de base ; et
c) le refroidissement de l'ensemble à température ambiante pour consolider le matériau élastomère thermoplastique et l'ouverture du moule ;
d) la fourniture d'une section supérieure du matériau élastomère thermoplastique avec des pores avant ou après l'ouverture du moule.

15. Procédé selon la revendication 14, dans lequel, après l'étape b), le moule est ouvert et des granulés supplémentaires de matériau élastomère thermoplastique sont ajoutés au moule, et l'étape b) est répétée.
